(19) Europäisches Patentamt / European Patent Office / Office européen des brevets

(11) **EP 1 495 755 B1**

(12) # FASCICULE DE BREVET EUROPEEN

(45) Date de publication et mention de la délivrance du brevet:
**14.10.2009 Bulletin 2009/42**

(51) Int Cl.:
***A61K 31/198** (2006.01)* ***A61P 1/14** (2006.01)*
***A61K 38/27** (2006.01)* ***A61K 38/30** (2006.01)*

(21) Numéro de dépôt: **04291738.5**

(22) Date de dépôt: **08.07.2004**

(54) **Utilisation de la citrulline dans la fabrication d'un médicament pour le traitement de l'insuffisance intestinale liée au vieillissement ou à une irradiation**

Verwendung von Citrullin zur Herstellung eines Arzneimittels zur Behandlung von ans Altern oder an eine Bestrahlung gebundener intestinaler Insuffizienz

Use of citrulline for the manufacture of a medicament in the treatment of intestinal failure linked to ageing or to an irradiation

(84) Etats contractants désignés:
**AT BE BG CH CY CZ DE DK EE ES FI FR GB GR HU IE IT LI LU MC NL PL PT RO SE SI SK TR**

(30) Priorité: **08.07.2003 FR 0308349**

(43) Date de publication de la demande:
**12.01.2005 Bulletin 2005/02**

(73) Titulaires:
- **Laboratoires Biocodex**
  **60200 Compiègne (FR)**
- **UNIVERSITE RENE DESCARTES (PARIS V)**
  **F-75270 Paris Cédex 06 (FR)**

(72) Inventeurs:
- **Osowska-Vincent, Sylwia**
  **F-75014 Paris (FR)**
- **Moinard, Christophe**
  **F-92340 Bourg La Reine (FR)**
- **Cynober, Luc**
  **F-92390 Sceaux (FR)**
- **Le Guern, Marie-Emmanuelle**
  **F-60200 Compiegne (FR)**
- **Hublot, Bernard**
  **F-60200 Compiegne (FR)**
- **Bernasconi, Paul**
  **F-75014 Paris (FR)**

(74) Mandataire: **Grosset-Fournier, Chantal Catherine**
**Grosset-Fournier & Demachy**
**54, Rue Saint-Lazare**
**75009 Paris (FR)**

(56) Documents cités:
**FR-A- 2 691 359** **US-A- 5 189 025**
**US-A1- 2001 056 068**

- **CRENN P ET AL: "Postabsorptive plasma citrulline concentration is a marker of absorptive enterocyte mass and intestinal failure in humans" GASTROENTEROLOGY 2000 UNITED STATES, vol. 119, no. 6, 2000, pages 1496-1505, XP002272145 ISSN: 0016-5085**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet l'utilisation de citrulline pour la préparation d'un médicament destiné au traitement de pathologies liées à l'insuffisance intestinale , les dites pathologies étant choisies dans le groupe comprenant l'insuffisance intestinale liée au vieillissement ou à une irradiation.

L'insuffisance intestinale est définie comme étant une diminution de la masse intestinale fonctionnelle en deçà de la quantité nécessaire à l'absorption des nutriments (Fleming et Remington, 1981). Elle se caractérise notamment par un défaut d'absorption digestive des graisses, des protéines, des glucides, ou des vitamines ; on parle alors de malabsorption, dont les répercussions peuvent être une dénutrition et un amaigrissement.

Une des conséquences de la malabsorption est en particulier de limiter les apports azotés et notamment d'arginine. Il a ainsi été montré chez le rat qu'en cas de résection massive de l'intestin grêle (80%), l'arginine devenait un acide aminé essentiel (Wakabayashi *et al.,* 1994).

L'arginine est un acide aminé impliqué dans de nombreuses fonctions de l'organisme. Elle intervient en particulier dans la synthèse des protéines, du monoxyde d'azote, des polyamines, de la créatine et de la proline, de plus elle possède des effets de stimulation de la sécrétion des hormones, telles que l'insuline, la prolactine, le glucagon et l'hormone de croissance.

Il est donc crucial en cas d'insuffisance intestinale de rétablir un apport suffisant en arginine, tant les carences en arginine liées à une insuffisance intestinale ont des répercussions importantes sur l'organisme.

Le document US 2001/0056068 décrit une méthode de traitement de pathologies liées à une diminution du taux d'oxyde nitrique endogène, telles que l'altération de la motilité intestinale et la sténose du pylore, comprenant l'administration de citrulline en tant qu'agent augmentant le taux d'oxyde nitrique endogène. Toutefois, il n'est fait aucune mention d'un quelconque traitement de l'insuffisance intestinale dans ce document.

Ainsi, le but de la présente invention est de fournir un moyen de traiter les pathologies liées à l'insuffisance intestinale , les dites pathologies étant choisies dans le groupe comprenant l'insuffisance intestinale liée au vieillissement ou à une irradiation.

La présente invention découle notamment de la mise en évidence par les Inventeurs que l'administration par voie entérale de L-citrulline à des rats ayant subi une résection sévère de l'intestin grêle permettait d'augmenter le gain de poids, d'augmenter la concentration plasmatique en L-arginine et d'améliorer le bilan azoté, par rapport à des rats ayant subi une résection semblable mais n'ayant pas reçu de L-citrulline. De manière inattendue, l'administration de L-citrulline est plus efficace pour augmenter le gain de poids, augmenter la concentration plasmatique en L-arginine et améliorer le bilan azoté, que l'administration de L-arginine elle-même.

La présente invention concerne l'utilisation de L-citrulline (I) pour la préparation d'un médicament destiné au traitement de pathologies liées à une insuffisance intestinale, les dites pathologies étant choisies dans le groupe comprenant l'insuffisance intestinale liée au vieillissement ou à une irradiation.

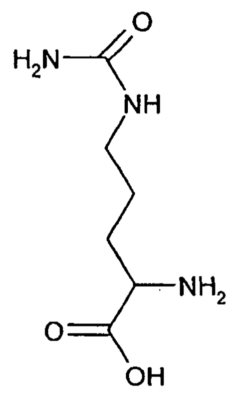

I

On désigne par « insuffisance intestinale » un état pathologique de l'intestin, notamment de l'intestin grêle, dans lequel l'absorption des nutriments est réduite par rapport à la normale, la diminution de l'absorption des nutriments étant liée à une diminution du nombre et/ou de la fonctionnalité de cellules intestinales capables d'assurer cette absorption, cette diminution du nombre et/ou de la fonctionnalité de cellules intestinales étant elle-même due soit à une élimination physique de ces cellules (notamment par chirurgie ou par radiations), soit à un dysfonctionnement pathologique de ces cellules.

A ce titre, il convient de noter que les altérations de la motilité intestinale, ainsi que la sténose du pylore ne font pas partie du champs de l'invention.

En effet, les altérations de la motilité intestinale sont présentes dans des pathologies fonctionnelles telles que la constipation, le syndrome de l'intestin irritable, ou les diarrhées fonctionnelles. Ces pathologies ne sont déterminées ni par une diminution du nombre de cellules intestinales capables d'assurer les fonctions d'absorption, ni par une dysfonction de ces cellules (Isselbacher *et al.,* 1995 ; Dapoigny *et al.,* 2003).

Par ailleurs, la sténose du pylore est une maladie relativement fréquente du nourrisson. Il s'agit d'une malformation congénitale dans laquelle le pylore est hypertrophié et constitue un obstacle mécanique au passage de l'alimentation. Son traitement est chirurgical, il s'agit d'une pyloromyotomie, simple et rapide, à l'issue de laquelle l'enfant peut reprendre très vite une alimentation normale. Il s'agit donc d'une pathologie sans rapport avec une insuffisance intestinale (Jacqmarcq *et al.,* 2004 ; Rambaud et Bouhnik, 2001).

Une des conséquences fréquentes de l'insuffisance intestinale est d'entraîner un état de dénutrition. L'utilisation de L-citrulline permet notamment d'améliorer l'état nutritionnel et pondéral d'individus souffrant d'insuffisance intestinale.

Les pathologies liées à l'insuffisance intestinale peuvent être:

- le syndrome du grêle court faisant suite à une résection intestinale, notamment dans les cas d'ischémie mésentérique aiguë, de thrombose de la veine mésentérique supérieure, de volvulus du grêle et de hernies étranglées, de pseudo-obstruction intestinale chronique, de grêle radique, de maladie de Crohn, de jéjuno-iléite ulcéreuse non granulomateuse, de traumatisme abdominal ; le syndrome du grêle court fait notamment suite à des résections de l'intestin grêle laissant au maximum 1 mètre d'intestin grêle en plus du duodénum ; ces résections entraînent en période postopératoire immédiate une insuffisance intestinale caractérisée par une malabsorption constante et majeure, parfois aggravée par une hypersécrétion gastrique, qui conduit à la mise en place d'une nutrition parentérale totale, rapidement associée à une nutrition entérale continue, puis à une alimentation orale ; l'adaptation de l'intestin restant est possible entre 2 et 6 mois après l'intervention, mais l'amélioration des capacités d'absorption de l'intestin grêle reste le plus souvent insuffisante (Rambaud et Bouhnik, 2001 ; Colombel et Dupas, 1997) ;
- la maladie coeliaque ; la maladie coeliaque est une entéropathie chronique caractérisée par une intolérance alimentaire au gluten, et plus particulièrement à des protéines contenues dans certaines céréales, telles que la gliadine, l'hordéine ou la sécaline ; cette maladie survient chez des sujets génétiquement prédisposés ; la muqueuse intestinale d'un patient atteint de maladie coeliaque est le siège d'un processus inflammatoire, de nature en partie immunitaire, qui provoque notamment une atrophie des villosités ; l'insuffisance intestinale résultante est caractérisée par une malabsorption intestinale, qui se manifeste par une diarrhée avec stéatorrhée, un amaigrissement et une dénutrition ; les conséquences biologiques de la malabsorption sont notamment une anémie associée à une carence en fer, en folate ou en vitamine B12, un déficit en facteurs de la coagulation dépendant de la vitamine K, une hypoprotidémie, une hypoalbuminémie, une hypocalcémie, une hypomagnésémie et un déficit en zinc (Cellier et Grosdidier, 2001 ; Jadoulle, 2002) ;
- les maladies inflammatoires chroniques de l'intestin, telles que la maladie de Crohn et la recto-colite ulcéreuse, aussi nommée recto-colite hémorragique ; ces maladies sont des maladies chroniques évoluant par poussées, elles sont souvent accompagnées, notamment dans le cas de la maladie de Crohn, d'une insuffisance intestinale chronique ayant pour cause des fistules, des obstacles mécaniques sur l'intestin grêle, ou une entéropathie non sténosante ; la réduction de la masse intestinale fonctionnelle qui s'ensuit ne permet pas le maintien de l'état nutritionnel ; il en résulte en effet une malnutrition protéino-énergétique, qui s'accompagne d'une malabsorption et d'une augmentation des pertes hydro-électrolytiques et d'éléments minéraux, tels que le calcium, le magnésium ou le zinc, ce qui conduit fréquemment à une dénutrition (Rambaud et Bouhnik, 2001 ; Crenn *et al.*, 2001) ;
- l'insuffisance intestinale liée au vieillissement ; la malnutrition protéino-énergétique est fréquente chez les personnes âgées ; en effet environ 40% des personnes âgées de plus de 70 ans sont concernées ; en situation de dénutrition le vieillissement est caractérisé par des modifications morphologiques et fonctionnelles de l'intestin grêle ; ces altérations peuvent conduire à une malabsorption et aggraver la dénutrition préexistante ; de plus cette dénutrition aggrave les altérations de l'appareil digestif liées à l'âge ; par ailleurs, la dégradation de l'état nutritionnel favorise les risques d'infections ; enfin, il est décrit que le sujet âgé dénutri présente un défaut de réponse à la réalimentation standard par rapport à ce qui est observé chez l'adulte ;
- l'insuffisance intestinale liée à une irradiation, aussi nommée grêle radique ; dans ce cas l'insuffisance intestinale est provoquée par l'irradiation de l'intestin grêle, en particulier dans le cas de radiothérapies, et plus particulièrement dans le cadre du traitement de cancers se développant dans la cavité abdomino-pelvienne.

Selon un autre mode de réalisation, la présente invention concerne l'utilisation susmentionnée de L-citrulline, pour la préparation d'une composition pharmaceutique comprenant, à titre de principe actif, de la L-citrulline, ou un sel

pharmaceutiquement acceptable de celle-ci, en association avec un véhicule pharmaceutiquement acceptable.

On désigne notamment par « sel pharmaceutiquement acceptable » des sels de citrulline tels que le malate de citrulline.

Les véhicules pharmaceutiquement acceptables apparaîtront de manière évidente à l'homme de l'art.

L'invention concerne notamment l'utilisation susmentionnée de L-citrulline, pour la préparation d'une composition pharmaceutique **caractérisée en ce que** la dose unitaire en L-citrulline est d'environ 1 g à environ 10 g, notamment environ 5 g, pour une posologie d'environ 0,05 g/kg/jour à environ 0,50 g/kg/jour, notamment environ 0,25 g/kg/jour.

L'invention concerne plus particulièrement l'utilisation susmentionnée de L-citrulline, pour la préparation d'une composition pharmaceutique se présentant sous forme sèche ou sous forme de solution aqueuse.

L'invention concerne plus particulièrement l'utilisation susmentionnée de L-citrulline, pour la préparation d'une composition pharmaceutique se présentant sous une forme administrable par voie orale, intrapéritonéale, entérale ou parentérale.

L'administration par voie entérale correspond notamment à une administration par sonde gastrique ou intestinale, l'administration par voie parentérale correspond notamment à une administration par perfusion intraveineuse centrale, périphérique ou sous-cutanée.

L'invention concerne plus particulièrement l'utilisation susmentionnée de L-citrulline, pour la préparation d'une composition pharmaceutique comprenant également un ou plusieurs autres composés destinés au traitement de l'insuffisance intestinale, tels que la glutamine, l'ornithine, l'hormone de croissance, ou la somatomédine C.

L'hormone de croissance, aussi nommée somatotrophine, est avantageusement sous forme recombinante, la somatomédine C, aussi nommée IGF-1 pour *insulin-like growth factor,* est également avantageusement sous forme recombinante.

Selon un autre mode de réalisation la présente invention concerne une composition pharmaceutique **caractérisée en ce qu**'elle comprend, à titre de principe actif, de la L-citrulline, ou un sel pharmaceutiquement acceptable de celle-ci, en association avec au moins un autre composé destiné au traitement de l'insuffisance intestinale, tels que les composés définis ci-dessus, et avec un véhicule pharmaceutiquement acceptable.

Selon un autre mode de réalisation l'invention concerne des produits comprenant :

- de la L-citrulline,
- et au moins un autre composé destiné au traitement de l'insuffisance intestinale, tels que les composés définis ci-dessus,

comme produits de combinaison pour une utilisation simultanée, séparée, ou étalée dans le temps, dans le cadre du traitement de l'insuffisance intestinale liée au vieillissement ou à une irradiation.

## Description des figures

### Figure 1

La Figure 1 représente le gain pondéral en grammes (en ordonnée) de rats ayant subi une résection intestinale et recevant une nutrition entérale standard (AANE), une nutrition entérale standard supplémentée en arginine (ARG), une nutrition entérale standard supplémentée en citrulline (CIT) ou n'ayant pas subi de résection (SHAM) après 10 jours de traitement. Chaque barre est surmontée de la représentation de l'estimation de l'erreur de mesure.

### Figure 2

La Figure 2 représente la concentration plasmatique en arginine en μmol/L (en ordonnée) de rats ayant subi une résection intestinale et recevant un nutrition entérale standard (AANE), une nutrition entérale standard supplémentée en arginine (ARG), une nutrition entérale standard supplémentée en citrulline (CIT) ou n'ayant pas subi de résection (SHAM) après 10 jours de traitement. Chaque barre est surmontée de la représentation de l'estimation de l'erreur de mesure.

### Figure 3

La Figure 3 représente le bilan azoté cumulé en grammes (en ordonnée) de rats ayant subi une résection intestinale et recevant une nutrition entérale standard (AANE, barre noire), une nutrition entérale standard supplémentée en arginine (ARG, barre hachurée verticalement), une nutrition entérale standard supplémentée en citrulline (CIT, barre hachurée obliquement) ou n'ayant pas subi de résection (SHAM, barre blanche), pour chacun des 10 jours du traitement, de 1 à 10 (en abscisse). Chaque barre est surmontée de la représentation de l'estimation de l'erreur de mesure.

**Figure 4**

La Figure 4 représente le contenu protéique du muscle Tibialis en mg/organe (en ordonnées) de rats âgés témoins (AL), de rats âgés dénutris (R), de rats âgés dénutris ayant reçu une alimentation supplémentée en acides aminés non essentiels (AANE) et de rats âgés dénutris ayant reçu une alimentation supplémentée en L-citrulline (CIT) (en abscisses). L'étoile indique que la valeur mesurée pour le groupe CIT est significativement différente de celles mesurées pour les groupes AL, AANE et R.

**Figure 5A et Figure 5B**

La Figure 5A représente la vitesse de synthèse protéique relative (FSR) dans le muscle Tibialis en pourcentage par heure (en ordonnées) pour des rats âgés témoins (AL), de rats âgés dénutris (R), de rats âgés dénutris ayant reçu une alimentation supplémentée en acides aminés non essentiels (AANE) et de rats âgés dénutris ayant reçu une alimentation supplémentée en L-citrulline (CIT) (en abscisses).

La Figure 5B représente la vitesse de synthèse protéique absolue (ASR) dans le muscle Tibialis en mg de protéines par heure (en ordonnées) de rats âgés témoins (AL), de rats âgés dénutris (R), de rats âgés dénutris ayant reçu une alimentation supplémentée en acides aminés non essentiels (AANE) et de rats âgés dénutris ayant reçu une alimentation supplémentée en L-citrulline (CIT) (en abscisses). L'étoile indique que la valeur mesurée pour le groupe CIT est significativement différente de celles mesurées pour les groupes AANE et R.

**Exemple 1** qui ne fait pas partie de l'invention

**Mise en évidence de l'effet d'une alimentation supplémentée en citrulline dans le cadre de l'insuffisance intestinale *appliquée à un modèle de syndrome du grêle court***

Le rat a été choisi comme modèle pour évaluer l'effet d'une supplémentation en citrulline dans le cadre d'une insuffisance intestinale et notamment dans le cadre du syndrome du grêle court.

24 rats Wistar d'environ 220-230 g ont été utilisés. Ils ont été acclimatés en cages métaboliques pendant 5 jours avant d'être opérés ; durant cette période ils avaient un accès libre à l'eau et à de la nourriture de laboratoire standard.

Les animaux ont ensuite été opérés : 18 d'entre eux ont subi une résection intestinale d'environ 80% de la longueur totale de l'intestin grêle, les 6 derniers rats ont été opérés mais n'ont pas subi de résection.

4 groupes d'animaux ont été constitués :

- un groupe AANE, formé de 6 animaux ayant subi une résection et recevant une nutrition entérale standard (Sondalis®, Nestlé Clinical Nutrition),
- un groupe ARG, formé de 6 animaux ayant subi une résection et recevant une nutrition entérale standard supplémentée par une dose de L-arginine (Sigma-Aldrich) de 0,994 g/kg/jour ;
- un groupe CIT, formé de 6 animaux ayant subi une résection et recevant une nutrition entérale standard supplémentée par une dose de L-citrulline (Laboratoires Biocodex) de 1 g/kg/jour (i.e. en quantité équimolaire à l'arginine),
- un groupe SHAM témoin, formé de 6 animaux opérés mais n'ayant pas subi de résection, recevant une nutrition entérale standard.

Les apports en L-arginine et en L-citrulline correspondent à ceux utilisés chez l'homme en tenant compte de la différence de vitesse métabolique entre les deux espèces (vitesse environ 10 fois plus élevée chez le rat).

La nutrition entérale a été réalisée durant 10 jours en mode continu par la pose d'une sonde gastrique. L'apport azoté était de 2 g/kg/jour et l'apport calorique de 290 kcal/kg/jour. Les rations alimentaires ont été rendues iso-azotées par apport d'un mélange d'acides aminés non-essentiels (Gly, Ala, Ser, His, Pro, Asn) (Sigma-Aldrich).

Trois types de mesures ont notamment été réalisées : une mesure quotidienne du poids des animaux, une mesure de la concentration plasmatique en arginine au terme de l'expérience par chromatographie d'échange d'ions à l'aide d'un appareil JEOL et un suivi quotidien du bilan azoté cumulé en sommant les différences journalières entre la quantité d'azote absorbée et la quantité d'azote évacuée dans les urines par pyroluminescence à l'aide d'un appareil ANTEK.

Les résultats présentés dans la **Figure 1** indiquent que le gain de poids du groupe AANE au bout de 10 jours (environ 15 g) est inférieur à celui du groupe témoin SHAM (environ 21 g) ; en revanche le gain de poids du groupe ARG (environ 17 g) est supérieur à celui du groupe AANE mais inférieur à celui du groupe témoin SHAM, et le gain de poids du groupe CIT (environ 21 g) est équivalent à celui du groupe témoin SHAM. La supplémentation en citrulline permet donc de compenser la diminution de prise de poids due à la résection, ce qui indique qu'en cas d'insuffisance intestinale, l'administration de citrulline permet de maintenir l'état nutritionnel des animaux.

Les résultats présentés dans la **Figure 2** indiquent que la concentration plasmatique en arginine est abaissée pour le groupe AANE par rapport au groupe témoin SHAM, ce qui confirme les données de l'art antérieur concernant la carence en arginine consécutive à une résection intestinale sévère. En revanche une supplémentation en arginine (groupe ARG) permet de compenser cet effet, et même d'augmenter la concentration plasmatique en arginine par rapport au témoin. Cet effet est encore plus marqué dans le cas de la citrulline (groupe CIT).

Cependant, les résultats présentés dans la **Figure 3** indiquent que seule la supplémentation en citrulline (groupe CIT) permet de rétablir un bilan azoté des animaux ayant subi une résection, au même niveau que celui du groupe témoin (SHAM), alors que ce n'est pas le cas d'une supplémentation en arginine (groupe ARG).

Globalement ces résultats indiquent qu'en cas d'insuffisance intestinale, la citrulline peut (i) permettre de maintenir l'état nutritionnel, (ii) compenser les effets d'une dénutrition en arginine en restaurant un bilan azoté normal et (iii) être absorbée par l'intestin dans des quantités suffisantes pour pouvoir exercer ses effets.

## Exemple 2

**Mise en évidence de l'effet d'une alimentation supplémentée en citrulline dans le cadre de l'insuffisance intestinale liée au vieillissement.**

Le rat a été choisi comme modèle pour évaluer l'effet d'une supplémentation en L-citrulline dans le cadre du traitement d'une insuffisance intestinale liée au vieillissement.

Tous les réactifs chimiques utilisés proviennent de Sigma (Saint-Quentin-Fallavier, France). La L-citrulline a été fournie par les Laboratoires Biocodex (Compiègne, France).

Des rats mâles Sprague-Dawley (Charles River Laboratories, L'arbresles, France) âgés de 19 mois ont été placés dans des cages individuelles, en ambiance thermostatée (23° $\pm$ 1°C), et soumis à un cycle lumière-obscurité de 12h (obscurité de 8h à 20h). Leur acclimatation a été effectuée pendant 2 semaines, au cours desquelles la consommation alimentaire spontanée a été mesurée. Ils ont été nourris avec un régime standard (A04, UAR, Villemoisson-sur-Orge, France) contenant 17% de protéines, 3% de lipides, 59% de glucides et 21% d'eau, fibres, vitamines et minéraux. La prise alimentaire moyenne, durant cette période est de 34,4 g/ jour.

Après la période d'acclimatation, les rats ont été répartis en 4 groupes (cf. figure 5): un groupe contrôle formé de rats (n=10) nourris *ad libitum* (AL) pendant 12 semaines et 3 autres groupes soumis à une restriction alimentaire durant la même période, c'est à dire nourris avec seulement 50% des ingesta spontanés (soit 17,2 g), avec le régime standard (UAR A04).

A la fin de la période de restriction, les animaux (n=10) d'un groupe ont été sacrifiés (groupe R) et les rats des deux groupes restants ont été nourris pendant une semaine avec 90% des ingesta spontanés (soit 30,9 g), soit avec un régime enrichi en acides aminés non essentiels (groupe AANE : alanine, asparagine, glycine, sérine, histidine, et proline apportés de façon équimolaire) soit avec un régime enrichi en L-citrulline (5g/kg/j) (groupe citrulline). Les apports des deux groupes étaient isoazotés et isocaloriques. La limitation à 90 % des apports spontanés permet d'être certain que les rats consomment l'intégralité des aliments qui leur sont offerts (Walrand *et al.,* 2000).

Le poids corporel a été mesuré tous les trois jours tout au long de la période d'expérimentation.

A la fin de la période expérimentale, les rats, en situation postabsorptive, ont été anesthésiés avec de l'isoflurane (3%) et ont reçu une injection sous-cutanée de $^{13}$C-valine à des temps variables avant d'être tués par décapitation.

Les 2 muscles *Tibialis* (TIB) ont été prélevés pour déterminer d'une part le contenu en protéines et en acides aminés (TIB droit) et d'autre part, les vitesses de synthèse protéique fractionnaire (FSR) et absolue (ASR) (TIB gauche). Tous les tissus prélevés ont préalablement été pesés avant d'être congelés dans de l'azote liquide et stockés à -80°C jusqu'aux analyses finales.

Le sang a été recueilli sur héparine et centrifugé (3500 tr/min, à + 4°C durant 15 min). Puis, le plasma a été déprotéinisé à l'aide de 100 $\mu$L d'une solution d'acide sulfosalicylique à 30% pour 1 mL de plasma. Les échantillons ont été centrifugés (5000 tr/min, + 4°C, 5 min). Une fraction aliquote du surnageant a été dosée par chromatographie échangeuse de cations sur analyseur d'acides aminés (Jéol, Tokyo, Japon). Les résultats ont été exprimés en $\mu$mol/L.

La détermination du contenu protéique a été effectuée au niveau du TIB. Les tissus congelés ont été broyés et homogénéisés dans une solution d'acide trichloroacétique (ATC) à 10% (1mL pour 100 mg de tissus) à l'aide d'un broyeur Ultra-Turrax T25® (Ika Labotechnik, Staufer, Allemagne), en maintenant le tube dans la glace pilée. L'homogénat a ensuite été centrifugé 10 min à 3500 tr/min. Puis, le culot de broyage a été délipidé avec un mélange alcool éthylique/ éther (v/v), puis dissous dans la soude 1N (4 mL pour 100 mg de tissus) pendant 12h à 40°C. Le contenu du TIB en protéines est mesuré selon la méthode de Gomall. Les résultats sont exprimés en mg/muscle.

La détermination des teneurs en acides aminés a été effectuée au niveau du *Tibialis.* Pour ce faire, les acides aminés intra-tissulaires ont été extraits avant leur dosage. Pour cela, le muscle considéré a été broyé en présence d'une solution d'ATC (acide trichloroacétique) à 10% (1 ml pour 100 mg de tissu) dans un tube en verre plongé dans de la glace. Le broyage a été effectué avec un appareil Ultra-Turrax®, jusqu'à obtention d'une suspension homogène. Après

centrifugation (10 min à 3500 tr/min à +4°C), le surnageant, qui contient les acides aminés libres, a été réparti en fractions aliquotes et congelé à -80°C jusqu'au dosage. Celui-ci a été effectué par chromatographie échangeuse de cations, à l'aide d'un analyseur modèle Aminotac, Jéol, (Tokyo, Japon). Les résultats sont exprimés en nmol/g de tissu.

La vitesse de synthèse a été déterminée au niveau du TIB par la méthode d'injection d'une dose de charge d'un acide aminé traceur. Le principe de la technique repose sur la détection par spectrométrie de masse de l'incorporation de cet acide aminé traceur dans les protéines d'intérêt (Guillet *et al.*, 2004). En pratique, la $^{13}$C-Valine (150 $\mu$mol/100g de poids corporel, Cambridge Isotope Laboratories, MA, USA) a été injectée par voie sous-cutanée (3,6 mL pour 600 g de poids corporel). Puis, afin de prendre en compte la vitesse d'incorporation du traceur dans les protéines, les animaux d'un même groupe ont été tués à des temps différents : 10, 15, 20, 25, 30, 35, 40, 45, 50 et 55 min post injection. Néanmoins, les effectifs des groupes AL (n=8) et R (n=9) ne permettant pas d'intégrer tous les temps, il a fallu exclure 2 points expérimentaux dans le groupe AL (t=45 et 55 min) et 1 point dans le groupe R (t=55 min). Ceci a permis de déterminer l'incorporation du traceur dans les protéines d'intérêt et d'établir une droite d'incorporation (Breuille *et al.,* 1998). Après broyage de 100 mg de muscle, les protéines ont été extraites par précipitations successives à l'acide trichloracétique (ATC). Elle ont ensuite été hydrolysées par de l'HCl (6M, 110°C, 24h) et les acides aminés ainsi obtenus ont été purifiés par chromatographie échangeuse de cations (Dowex 50W 8X, Bio-Rad, Hercules, CA). Les acides aminés ont été élues par la soude (4M) et séchés au speedvac (Savant Instrument Inc, USA). Ils ont ensuite été transformés en dérivés N-acétyl-propyl. Par ailleurs, l'enrichissement en $^{13}$C-valine dans le compartiment des acides aminés libres musculaires a également été mesuré par spectrométrie de masse couplée à une chromatographie en phase gazeuse (type GC-MS, Hewlett-Packard 5971A, USA), après extraction des acides aminés par l'acide perchlorique et dérivation en esters de t-butyldiméthylsilyl.

La vitesse fractionnaire de synthèse des protéines myofibrillaires (FSR) exprimée en % par heure a été calculée pour chaque point en prenant en compte à la fois l'enrichissement en $^{13}$C-valine des protéines musculaires, mais également celui du compartiment précurseur de la synthèse (pool d'acides aminés libres musculaires). Ainsi, la formule suivante est utilisée :

$$\mathbf{FSR=[(Sb - Sb0) / (Sa \times t)] \times 100}$$

où :

- Sb : enrichissement des protéines musculaires en $^{13}$C-valine au temps t ;
- Sb0 : enrichissement naturel des protéines musculaires en $^{13}$C-valine ;
- Sa: enrichissement en $^{13}$C-valine dans le compartiment d'acides aminés libres intramusculaires ;
- t : temps post-injection de prélèvement du muscle en heure.

Ce paramètre est également exprimé en quantité absolue de protéines musculaires synthétisées par heure (ASR, en mg/h) en prenant en compte la quantité totale de protéines au sein des TIB de chaque rat, selon l'équation :

$$\mathbf{ASR=[FSR \times \text{quantité de protéine en mg}]/100}$$

Les résultats sont présentés sous la forme de moyenne $\pm$ écart standard à la moyenne (ESM). Les résultats sont analysés par une ANOVA suivie d'un test *a posteriori* de Duncan. Le logiciel PCSM est utilisé (Deltosoft, Grenoble, France). Les valeurs de $p<0.05$ sont considérées comme significatives.

Les résultats obtenus indiquent que seule la supplémentation en L-citrulline augmente significativement les concentrations plasmatiques et musculaires en CIT, ORN et ARG (CIT *vs* AL, R, AANE, $p<0.05$) (Tableau 1).

**Tableau 1 : Concentrations en acides aminés dans le plasma et le TIB**

| Plasma | | | | |
|---|---|---|---|---|
| | **AL** | **R** | **AANE** | **CIT** |
| **Citrulline** | 116 ± 7 | 104 ± 7 | 155 ± 20 | 2394 ± 279* |
| **Ornithine** | 46 ± 3 | 41 ± 3 | 43 ± 6 | 223 ± 27* |

(suite)

| Plasma | | | | |
|---|---|---|---|---|
| | **AL** | **R** | **AANE** | **CIT** |
| **Arginine** | 114 ± 9 | 118 ± 4 | 99 ± 7 | 561 ± 55* |
| **TIB** | | | | |
| | **AL** | **R** | **AANE** | **CIT** |
| **Citrulline** | 23 ± 4 | 26 ± 2 | 50 ± 11 | 616 ± 104* |
| **Ornithine** | 4 ± 0 | 6 ± 0 | 3 ± 0 | 20 ± 4* |
| **Arginine** | 29 ± 3 | 66 ± 4 | 22 ± 3 | 203 ± 35* |
| MOYENNE ±ESM. Les résultats sont exprimés en µmol/L et nmol/g respectivement<br>ANOVA+ test de Duncan : *p<0,05 *vs* AL, R et AANE | | | | |

Par ailleurs, la restriction alimentaire n'induit pas de diminution significative du contenu protéique musculaire. Toutefois, à la suite de la phase de renutrition, une augmentation significative des concentrations protéiques du TIB est observée uniquement pour le groupe CIT (CIT *vs* AL, R, AANE, p<0,05) **(Figure 4).**

Enfin, bien que supérieure pour la L-citrulline par rapport aux autres groupes, la variation de la FSR observée ne semble pas significative **(Figure 5A).** En revanche, il y a une augmentation significative de l'ASR uniquement pour le groupe recevant une nutrition enrichie en L-citrulline (CIT *vs* R et AANE, p<0,05) **(Figure 5B).**

Par conséquent, la supplémentation en L-citrulline du régime alimentaire de rats âgés dénutris permet d'obtenir une augmentation significative de la masse musculaire et de l'accrétion protéique, notamment au niveau du *Tibialis*. Ceci indique que la L-citrulline est adaptée au traitement de l'insuffisance intestinale liée au vieillissement.

**REFERENCES**

Breuille D et al. Clin.Sci. (1998) 94 : 413-423

Cellier C et Grosdidier E. Maladie Coeliaque de l'Adulte. Rev. Prat. (2001) 51 : 959-963.

Colombel JF et Dupas JL. Pathologie du Grêle. In : Progrès en Hépato-gastroentérologie. Paris, Doin, Ed. (1997) 219-220.

Crenn P et al. Insuffisance Intestinale. Rev. Prat. (2001) 51 : 977-982.

Dapoigny et al. Troubles fonctionnels intestinaux et consommation de soins. Gastroentérologie Clinique et Biologique, (2003) 27 : 265-271.

Fleming CR et Remington M. Intestinal Failure. In : Nutrition and the Surgical Patient : Clinical Surgery International. Hill GI, Ed. Edimbourg : Churchill Livingstone, (1981) 219-235.

Guillet C et al. Exp. Gerontol. (2004) 39 : 745-751

Isselbacher KJ et al. Trouble de la motricité intestinale. In: Harrison Médecine Interne. Arnette Blackwell S.A. (13ème édition) (1995) 1418-1422.

Jacqmarcq et al. Prise en charge périopératoire de la sténose du pylore en France en 1999. Annales françaises d'Anesthésie et de Réanimation. (2004) 23 : 31-38.

Jadoulle J. Interactions Somato-Psychiques dans la Maladie Coeliaque. Gastroentérol. Clin. Biol. (2002) 26 : 1134-1139.

Wakabayashi Y et al. Arginine Becomes an Essential Amino Acid after Massive Resection of Rat Small Intestine. J. Biol. Chem. (1994) 269 : 32667-32671.

Rambaud JC et Bouhnik Y. Intestin Grêle. In : Le Livre de l'Interne en Gastroentérologie. Paris : 2ème édition, Flamarion, Ed. (2001) 286-298, 533-536.

Rambaud JC et Bouhnik Y. sténose hypertrophique du pylore. In : Le Livre de l'Interne en Gastroentérologie. Paris : 2ème édition, Flamarion, Ed. (2001) 202.

Walrand S et al. Am.J.Clin.Nutr. (2001) 74 : 670-678

## Revendications

**1.** Utilisation de L-citrulline (I)

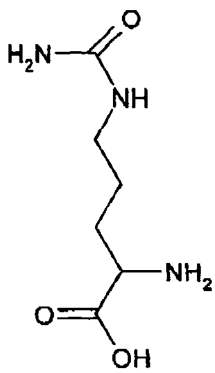

I

pour la préparation d'un médicament destiné au traitement de pathologies liées à une insuffisance intestinale, lesdites pathologies étant choisies dans le groupe comprenant l'insuffisance intestinale liée au vieillissement et l'insuffisance intestinale liée à une irradiation.

**2.** Utilisation de L-citrulline selon la revendication 1, pour la préparation d'une composition pharmaceutique comprenant, à titre de principe actif, de la L-citrulline, ou un sel pharmaceutiquement acceptable de celle-ci, en association avec un véhicule pharmaceutiquement acceptable.

**3.** Utilisation de L-citrulline selon l'une des revendications 1ou 2, pour la préparation d'une composition pharmaceutique **caractérisée en ce que** la dose unitaire en L-citrulline est d'environ 1 g à environ 10 g, notamment environ 5 g, pour une posologie d'environ 0,05 g/kg/jour à environ 0,50 g/kg/jour, notamment environ 0,25 g/kg/jour.

**4.** Utilisation de L-citrulline selon l'une des revendications 1 à 3, pour la préparation d'une composition pharmaceutique se présentant sous forme sèche ou sous forme de solution aqueuse.

**5.** Utilisation de L-citrulline selon l'une des revendications 1 à 4, pour la préparation d'une composition pharmaceutique se présentant sous une forme administrable par voie orale, intrapéritonéale, entérale ou parentérale.

**6.** Utilisation de L-citrulline selon l'une des revendications 1 à 5 pour la préparation d'une composition pharmaceutique comprenant également un ou plusieurs autres composés destinés au traitement de l'insuffisance intestinale, choisi (s) dans le groupe comprenant la glutamine, l'ornithine, l'hormone de croissance, ou la somatomédine C.

**7.** L-citrulline (I)

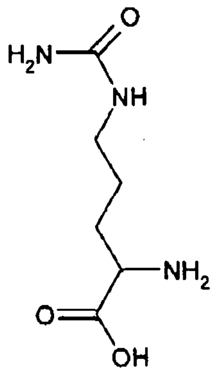

I

pour son utilisation dans le traitement de pathologies liées à une insuffisance intestinale, lesdites pathologies étant choisies dans le groupe comprenant l'insuffisance intestinale liée au vieillissement et l'insuffisance intestinale liée à une irradiation.

8. L-citrulline pour son utilisation selon la revendication 7, **caractérisée en ce qu'**on utilise la L-citrulline, ou un sel pharmaceutiquement acceptable de celle-ci, en association avec un véhicule pharmaceutiquement acceptable.

9. L-citrulline pour son utilisation selon l'une des revendications 7 ou 8, **caractérisée en ce qu'**on utilise la L-citrulline pour la préparation d'une composition pharmaceutique comprenant la dose unitaire en L-citrulline d'environ 1 g à environ 10 g, notamment environ 5 g, pour une posologie d'environ 0,05 g/kg/jour à environ 0,50 g/kg/jour, notamment environ 0,25 g/kg/jour.

10. L-citrulline pour son utilisation selon l'une des revendications 7 à 9 **caractérisée en ce qu'**on utilise la L-citrulline pour la préparation d'une composition pharmaceutique se présentant sous forme sèche ou sous forme de solution aqueuse.

11. L-citrulline pour son utilisation selon l'une des revendications 7 à 10 **caractérisée en ce qu'**on utilise la L-citrulline pour la préparation d'une composition pharmaceutique se présentant sous une forme administrable par voie orale, intrapéritonéale, entérale ou parentérale.

12. L-citrulline pour son utilisation selon l'une des revendications 7 à 11 **caractérisée en ce qu'**on utilise la L-citrulline pour la préparation d'une composition pharmaceutique comprenant également un ou plusieurs autres composés destinés au traitement de l'insuffisance intestinale, choisi(s) dans le groupe comprenant la glutamine, l'ornithine, l'hormone de croissance, ou la somatomédine C.

## Claims

1. Use of L-citrulline (I):

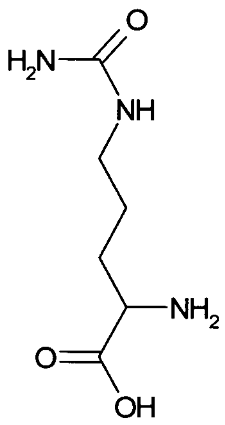

(I)

for the preparation of a drug for the treatment of pathologies linked to an intestinal insufficiency, said pathologies being selected from the group comprising intestinal insufficiency linked to ageing, and intestinal insufficiency linked to irradiation.

2. Use of L-citrulline according to claim 1 for the preparation of a pharmaceutical composition comprising as active substance, L-citrulline, or a pharmaceutically acceptable salt thereof, in combination with a pharmaceutically acceptable vehicle.

3. Use of L-citrulline according to claim 1 or 2 for the preparation of a pharmaceutical composition wherein the unit L-citrulline dose is approximately 1 g to approximately 10 g, in particular approximately 5 g, for a dosage of approximately 0.05 g/kg/day to approximately 0.50 g/kg/day, in particular approximately 0.25 g/kg/day.

4. Use of L-citrulline according to anyone of claim 1 to 3 for the preparation of a pharmaceutical composition wherein L-citrulline is presented in dry form or in the form of an aqueous solution.

5. Use of L-citrulline according to anyone of claim 1 to 4 for the preparation of a pharmaceutical composition wherein L-citrulline is presented in a form which can be administered by the oral, intraperitoneal, enteral or parenteral route.

6. Use of L-citrulline according to anyone of claim 1 to 5 for the preparation of a pharmaceutical composition also comprising one or more other compounds for the treatment of intestinal insufficiency chosen from the group comprising glutamine, ornithine, growth hormone, and somatomedin C.

7. L-citrulline (I):

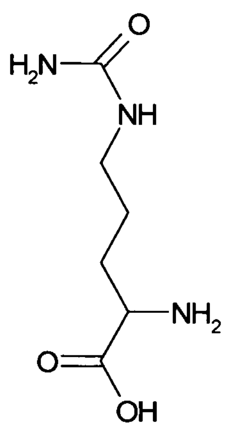

(I)

for its use for the treatment of pathologies linked to an intestinal insufficiency, said pathologies being selected from the group comprising intestinal insufficiency linked to ageing, and intestinal insufficiency linked to irradiation.

**8.** L-citrulline for its use according to claim 7 wherein L-citrulline, or a pharmaceutically acceptable salt thereof, are used in combination with a pharmaceutically acceptable vehicle.

**9.** L-citrulline for its use according to claim 7 or 8 wherein L-citrulline is used for the preparation of a pharmaceutical composition comprising the unit L-citrulline dose of approximately 1 g to approximately 10 g, in particular approximately 5 g, for a dosage of approximately 0.05 g/kg/day to approximately 0.50 g/kg/day, in particular approximately 0.25 g/kg/day.

**10.** L-citrulline for its use according to anyone of claims 7 to 9 wherein L-citrulline is used for the preparation of a pharmaceutical composition presented in dry form or in the form of an aqueous solution.

**11.** L-citrulline for its use according to anyone of claims 7 to 10 wherein L-citrulline is used for the preparation of a pharmaceutical composition presented in a form which can be administered by the oral, intraperitoneal, enteral or parenteral route.

**12.** L-citrulline for its use according to anyone of claims 7 to 11 wherein L-citrulline is used for the preparation of a pharmaceutical composition also comprising one or more other compounds for the treatment of intestinal insufficiency chosen from the group comprising glutamine, ornithine, growth hormone, and somatomedin C.

**Patentansprüche**

**1.** Verwendung von L-Citrullin (I)

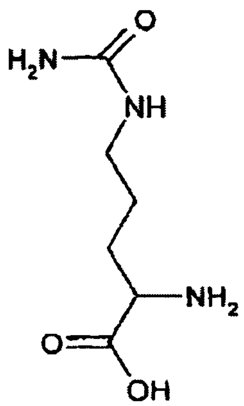

I

zur Herstellung eines Medikaments, das zur Behandlung von Pathologien bestimmt ist, die mit einer Darminsuffizienz verbunden sind, wobei die Pathologien aus der Gruppe ausgewählt sind, die die Darminsuffizienz, die mit dem Altern verbunden ist, und die Darminsuffizienz, die mit einer Bestrahlung verbunden ist, umfasst.

**2.** Verwendung von L-Citrullin nach Anspruch 1 zur Herstellung einer pharmazeutischen Zusammensetzung, die als Wirkstoff L-Citrullin oder ein pharmazeutisch verträgliches Salz davon in Verbindung mit einem pharmazeutisch verträglichen Vehikel umfasst.

**3.** Verwendung von L-Citrullin nach einem der Ansprüche 1 oder 2 zur Herstellung einer pharmazeutischen Zusammensetzung, **dadurch gekennzeichnet, dass** die Dosiseinheit von L-Citrullin etwa 1 g bis etwa 10 g, insbesondere etwa 5 g für eine Dosierung von etwa 0,05 g/kg/Tag bis etwa 0,50 g/kg/Tag, insbesondere etwa 0,25 g/kg/Tag beträgt.

**4.** Verwendung von L-Citrullin nach einem der Ansprüche 1 bis 3 zur Herstellung einer pharmazeutischen Zusammensetzung, die in trockener Form oder in Form einer wässrigen Lösung vorliegt.

**5.** Verwendung von L-Citrullin nach einem der Ansprüche 1 bis 4 zur Herstellung einer pharmazeutischen Zusammensetzung, die in einer Form vorliegt, die auf oralem, intraperitonealem, enteralem oder parenteralem Weg verabreichbar ist.

**6.** Verwendung von L-Citrullin nach einem der Ansprüche 1 bis 5 zur Herstellung einer pharmazeutischen Zusammensetzung, die außerdem eine oder mehrere andere Verbindungen umfasst, die zur Behandlung der Darminsuffizienz bestimmt sind, die aus der Gruppe ausgewählt (ist) sind, die Glutamin, Ornithin, Wachstumshormon oder Somatomedin C umfasst.

**7.** L-Citrullin (I)

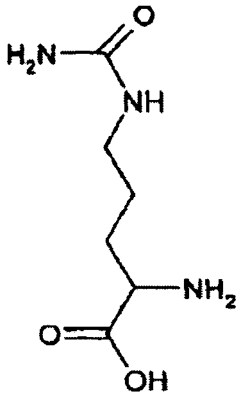

I

zur Verwendung bei der Behandlung von Pathologien, die mit einer Darminsuffizienz verbunden sind, wobei die Pathologien aus der Gruppe ausgewählt sind, die die Darminsuffizienz, die mit dem Altern verbunden ist, und die Darminsuffizienz, die mit einer Bestrahlung verbunden ist, umfasst.

**8.** L-Citrullin zur Verwendung nach Anspruch 7, **dadurch gekennzeichnet, dass** das L-Citrullin oder ein pharmazeutisch verträgliches Salz davon in Verbindung mit einem pharmazeutisch verträglichen Vehikel verwendet wird.

**9.** L-Citrullin zur Verwendung nach einem der Ansprüche 7 oder 8, **dadurch gekennzeichnet, dass** das L-Citrullin zur Herstellung einer pharmazeutischen Zusammensetzung verwendet wird, die die Dosiseinheit von L-Citrullin von etwa 1 g bis etwa 10 g, insbesondere etwa 5 g, für eine Dosierung von etwa 0,05 g/kg/Tag bis etwa 0,50 g/kg/Tag, insbesondere etwa 0,25 g/kg/Tag umfasst.

**10.** L-Citrullin zur Verwendung nach einem der Ansprüche 7 bis 9, **dadurch gekennzeichnet, dass** das L-Citrullin zur Herstellung einer pharmazeutischen Zusammensetzung verwendet wird, die in trockener Form oder in Form einer wässrigen Lösung vorliegt.

**11.** L-Citrullin zur Verwendung nach einem der Ansprüche 7 bis 10, **dadurch gekennzeichnet, dass** das L-Citrullin zur Herstellung einer pharmazeutischen Zusammensetzung verwendet wird, die in einer Form vorliegt, die auf oralem, intraperitonealem, enteralem oder parenteralem Weg verabreichbar ist.

**12.** L-Citrullin zur Verwendung nach einem der Ansprüche 7 bis 11, **dadurch gekennzeichnet, dass** das L-Citrullin zur Herstellung einer pharmazeutischen Zusammensetzung verwendet wird, die außerdem eine oder mehrere andere Verbindungen umfasst, die zur Behandlung der Darminsuffizienz bestimmt sind, die aus der Gruppe ausgewählt ist (sind), die Glutamin, Ornithin, Wachstumshormon oder Somatomedin C umfasst.

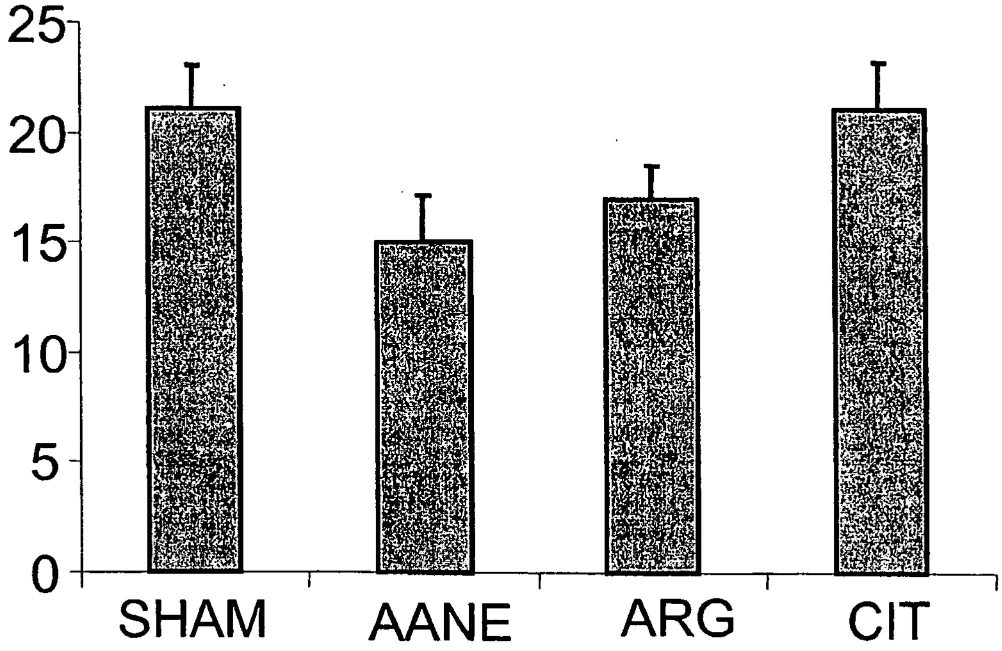


Figure 1

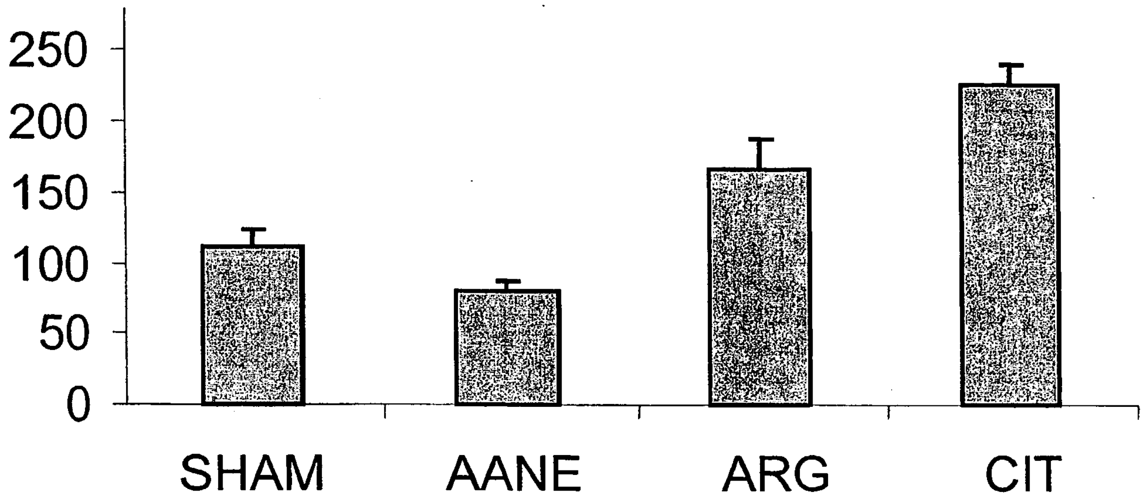


Figure 2

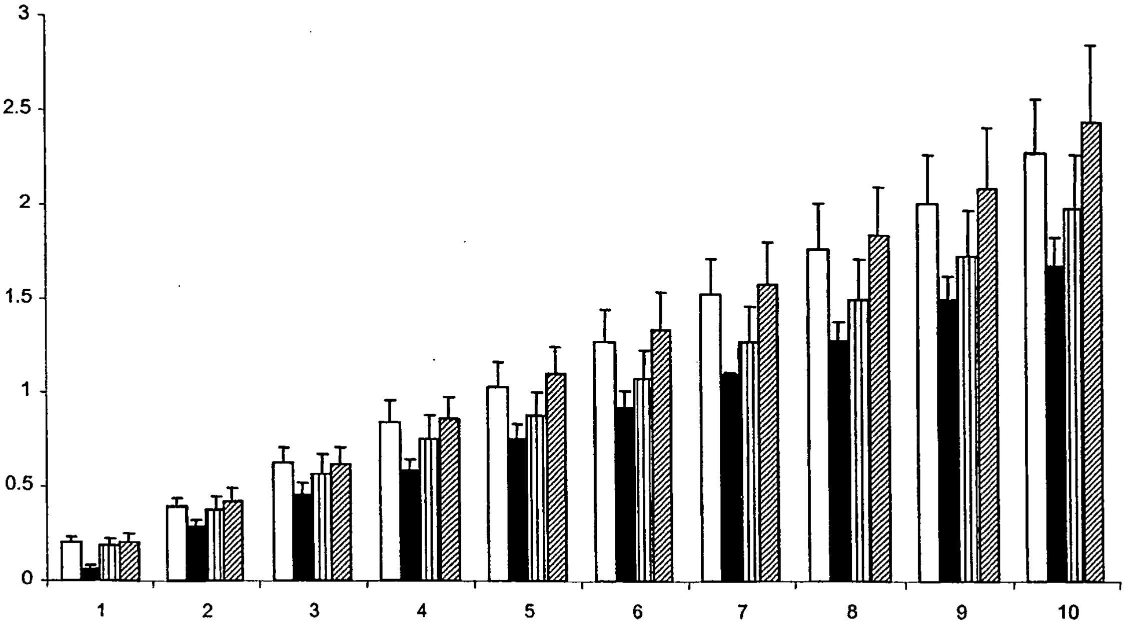


Figure 3

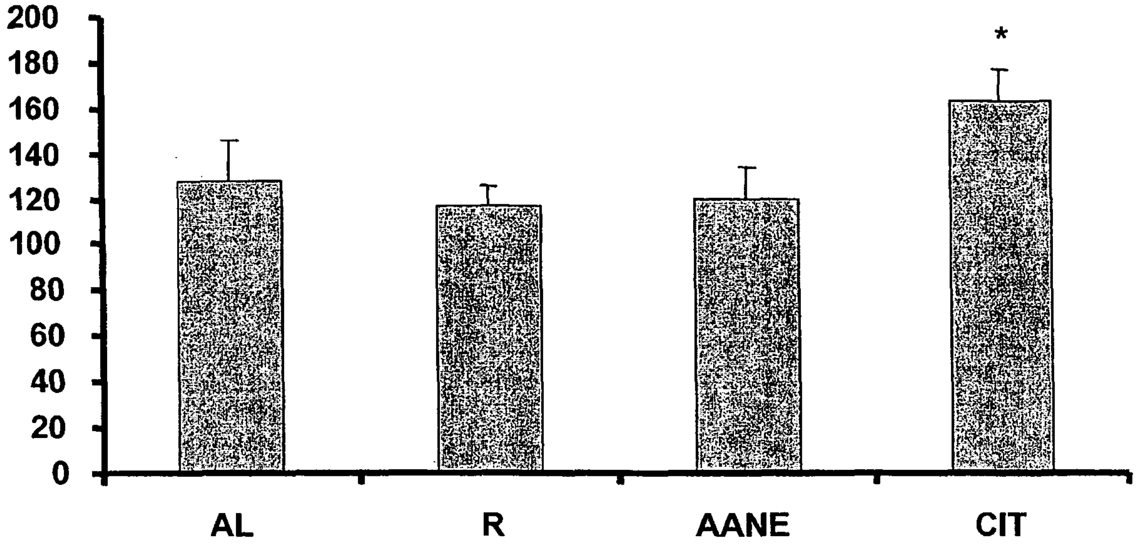


Figure 4

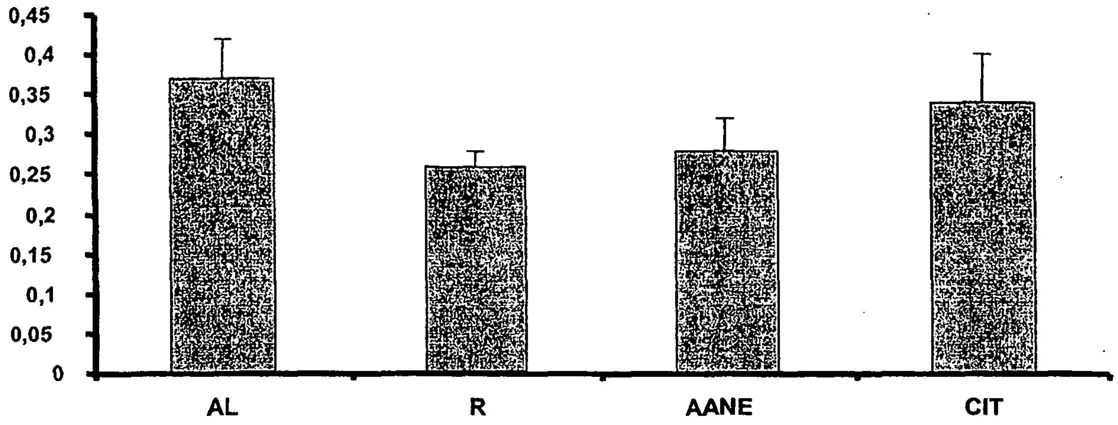


Figure 5A

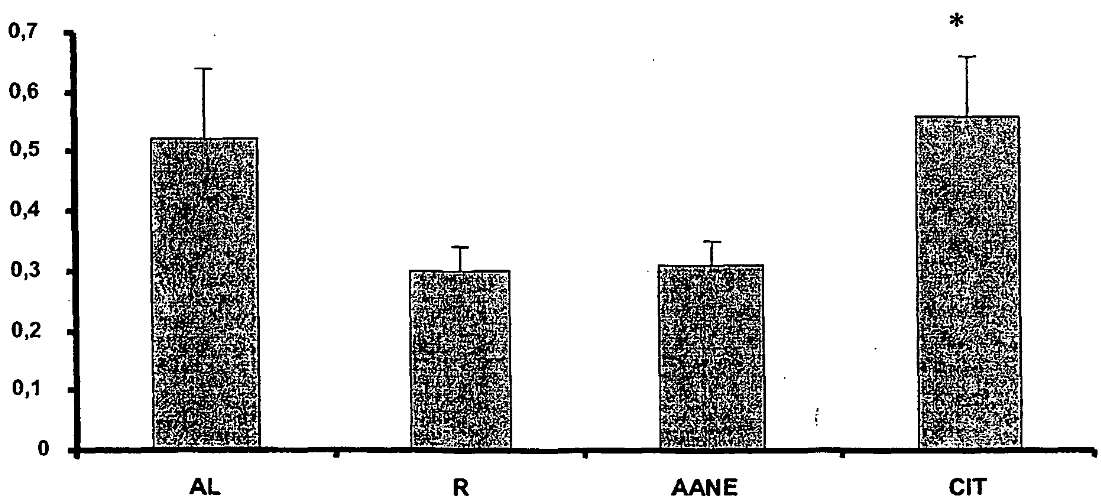


Figure 5B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20010056068 A


### Littérature non-brevet citée dans la description


- **Breuille D et al.** *Clin.Sci.,* 1998, vol. 94, 413-423
- **Cellier C ; Grosdidier E.** Maladie Coeliaque de l'Adulte. *Rev. Prat.,* 2001, vol. 51, 959-963
- Pathologie du Grêle. **Colombel JF ; Dupas JL.** Progrès en Hépato-gastroentérologie. 1997, 219-220
- **Crenn P et al.** Insuffisance Intestinale. *Rev. Prat.,* 2001, vol. 51, 977-982
- **Dapoigny et al.** Troubles fonctionnels intestinaux et consommation de soins. *Gastroentérologie Clinique et Biologique,* 2003, vol. 27, 265-271
- Intestinal Failure. **Fleming CR ; Remington M.** Nutrition and the Surgical Patient : Clinical Surgery International. 1981, 219-235
- **Guillet C et al.** *Exp. Gerontol.,* 2004, vol. 39, 745-751
- Trouble de la motricité intestinale. **Isselbacher KJ et al.** Harrison Médecine Interne. Arnette Blackwell S.A, 1995, 1418-1422
- **Jacqmarcq et al.** Prise en charge périopératoire de la sténose du pylore en France en 1999. *Annales françaises d'Anesthésie et de Réanimation,* 2004, vol. 23, 31-38
- **Jadoulle J.** Interactions Somato-Psychiques dans la Maladie Coeliaque. *Gastroentérol. Clin. Biol.,* 2002, vol. 26, 1134-1139
- **Wakabayashi Y et al.** Arginine Becomes an Essential Amino Acid after Massive Resection of Rat Small Intestine. *J. Biol. Chem.,* 1994, vol. 269, 32667-32671
- Intestin Grêle. **Rambaud JC ; Bouhnik Y.** Le Livre de l'Interne en Gastroentérologie. 2001, 286-298533-536
- **Rambaud JC ; Bouhnik Y.** Le Livre de l'Interne en Gastroentérologie. 2001, 202
- **Walrand S et al.** *Am.J.Clin.Nutr.,* 2001, vol. 74, 670-678